# EUROPÄISCHE PATENTSCHRIFT

(11) **EP 1 215 188 B1**
(45) Veröffentlichungstag und Bekanntmachung des Hinweises auf die Patenterteilung: **26.11.2003**
(21) Anmeldenummer: 01120749.5
(22) Anmeldetag: 06.09.2001
(51) Int. Cl.: C07B 41/06, C07C 45/51

(54) **Verfahren zur Herstellung von Carbonylverbindungen durch Spaltung von Hydroxycarbonsäuren**
Process for the preparation of carbonyl compounds by cleavage of hydroxycarboxylic acids
Procédé pour la préparation de composés carbonyls par clivage d'acides hydroxycarboxyliques

(30) Priorität: 14.12.2000 DE 10062178
(43) Veröffentlichungstag der Anmeldung: 19.06.2002
(73) Patentinhaber: Consortium für elektrochemische Industrie GmbH, 81379 München (DE)
(72) Erfinder: Sorger, Klas, Dr., 81371 München (DE); Petersen, Hermann, Dr., 84489 Burghausen (DE); Stohrer, Jürgen, Dr., 82049 Pullach (DE)
(74) Vertreter: Fritz, Helmut, Dr.

(56) Entgegenhaltungen:
- WO-A-96/15142
- C. S. RONDESTVEDT: "The base-catalyzed cleavage of beta-hydroxy acids" JOURNAL OF THE AMERICAN CHEMICAL SOCIETY, Bd. 78, Nr. 15, August 1956 (1956-08), Seiten 3804-3811, XP002185350 DC US
- M. D. IVANOFF: "Dédoublement alcalin des acides beta-hydroxyaliphatiques. III. Acides beta-lactiques disubstitués" BULLETIN DE LA SOCIETE CHIMIQUE DE FRANCE, Nr. 36, 1933, Seiten 321-323, XP001041229 PARIS FR

## Beschreibung

Die Erfindung betrifft ein Verfahren zur Herstellung von Carbonylverbindungen durch Spaltung von Hydroxycarbonsäuren in Gegenwart von Brönstedt-Base und nicht-hydroxylischem Lösungsmittel.

Durch Retroaldolisierung werden üblicherweise β-Hydroxycarbonsäureester, -amide oder β-Hydroxyketone thermisch, säure- oder basenkatalysiert in die entsprechenden Carbonylverbindungen gespalten (wie z. B. in WO 9615142).

Beispielsweise ist aus D. Ivanoff, Bull. Soc. Chim. France 36, 321 (1933) und C. S. Rondestvedt, M. E. Rowley, J. Am. Chem. Soc. 78, 3804 (1956) die Spaltung von di-, tri- und tetrasubstituierten β-Hydroxypropionsäuren in Wasser in Gegenwart der Basen Kaliumhydroxid oder -carbonat und in wäßriger alkoholischer Lösung in Gegenwart der Base Natriumhydroxid sowie in 1-Propanol in Gegenwart der Base Natrium-1-propanolat bekannt.

Die vorbekannten Verfahren haben folgende Nachteile:
Die Spaltung der β-Hydroxypropionsäuren verläuft in den meisten Fällen bei schlechten Gewichtsausbeuten unvollständig. Nur für wenige Fälle, wenn die β-Hydroxypropionsäuren über Arylgruppen in α- und/oder β-Stellung verfügen, verläuft die Spaltung vollständig. In einigen Fällen, insbesondere, wenn die β-Hydroxypropionsäuren Wasserstoffatome als Substituenten in α-und/oder β-Stellung besitzen, tritt keine Spaltung auf. Die Gewichtsausbeute, bezogen auf die bei der Spaltung gebildeten Carbonylverbindungen, ist deshalb in den meisten Fällen notwendigerweise reduziert.
Ferner verläuft die Spaltung der β-Hydroxypropionsäuren in den meisten Fällen sehr langsam und erfordert meist Reaktionszeiten von mehr als 10 h, was die Reaktion insbesondere bei Durchführung im technischen Maßstab aufgrund der schlechten Zeitausbeuten sehr unwirtschaftlich macht.
Zudem löst sich das mit Wasser mischbare Lösungsmittel 1-Propanol oder andere Alkohole bei wäßriger Aufarbeitung zur Gewinnung bzw. Isolierung der Carbonylverbindungen der allgemeinen Formel (1) in der wäßrigen Phase. Aus Gründen der Wirtschaftlichkeit und zur Verringerung der Abfallmengen ist insbesondere bei Anwendung im technischen Maßstab eine Rückgewinnung des eingesetzten Lösungsmittels aus der wäßrigen Phase (z. B. durch Extraktion oder Destillation) erforderlich, was allerdings mit erheblichem Aufwand verbunden ist.
Zusätzlich ist bei Verwendung des mit Wasser mischbaren Lösungsmittels 1-Propanol oder anderer Alkohole bei Aufarbeitung zur Gewinnung bzw. Isolierung der Carbonylverbindungen der allgemeinen Formel (1) üblicherweise der Einsatz von mit Wasser nicht mischbaren organischen Lösungsmitteln wie Ethylacetat oder Methyl-tert.-butylether als Cosolvenzien zur besseren Phasentrennung notwendig. Diese Lösungsmittel müssen zurückgewonnen und vor Wiederverwendung durch Destillation von Verunreinigungen befreit werden, was ebenfalls mit hohem Aufwand verbunden ist.

Es bestand daher die Aufgabe, ein ökonomisches Verfahren zu entwickeln, das es erlaubt, β-Hydroxycarbonsäuren zu spalten und die bei der Spaltung gebildeten Carbonylverbindungen in hohen Ausbeuten und Reinheiten zu gewinnen und zu isolieren.

Gegenstand der Erfindung ist ein Verfahren zur Herstellung von Carbonylverbindung der allgemeinen Formel (1)

R¹R²C=O (1),

bei dem β-Hydroxycarbonsäure oder deren Salz der allgemeinen Formel (2)

R¹R²C(OH)-CR³R⁴-COOM (2),

in Gegenwart von Brönstedt-Base, die ausgewählt wird aus Hydroxiden, Alkanolaten, Oxiden, Amiden und Hydriden der Alkali- und Erdalkalimetalle und nicht-hydroxylischem Lösungsmittel gespalten wird, wobei
- **R**^{**1**}: einen gegebenenfalls halogen- oder cyanosubstituierten C₁-C₃₀-Kohlenwasserstoffrest, in dem eine oder mehrere, einander nicht benachbarte Methyleneinheiten durch Gruppen -O-, -CO-, -COO-, -OCO-, oder -OCOO-, -S- oder -N**R**^{**x**}-ersetzt sein können und in denen eine oder mehrere, einander nicht benachbarte Methineinheiten durch Gruppen, -N=,-N=N-,oder -P= ersetzt sein können,
- **R**^{**2**}: Wasserstoff oder einen gegebenenfalls halogen- oder cyanosubstituierten C₁-C₃₀-Kohlenwasserstoffrest, in dem eine oder mehrere, einander nicht benachbarte Methyleneinheiten durch Gruppen -O-, -CO-, -COO-, -OCO-, oder -OCOO-, -S- oder -NR^{x}- ersetzt sein können und in denen eine oder mehrere, einander nicht benachbarte Methineinheiten durch Gruppen, -N=,-N=N-,oder -P= ersetzt sein können,
- R³ und R⁴: Wasserstoff, Halogen oder einen gegebenenfalls halogen- oder cyanosubstituierten C₁-C₃₀-Kohlenwasserstoff-rest, in dem eine oder mehrere, einander nicht benachbarte Methyleneinheiten durch Gruppen -O-, -CO-, -COO-, -OCO-, oder -OCOO-, -S- oder -N**R**^{**x**}- ersetzt sein können und in denen eine oder mehrere, einander nicht benachbarte Methineinheiten durch Gruppen, -N= , -N=N- , oder -P= ersetzt sein können,
- **R**^{**x**}: Wasserstoff oder einen gegebenenfalls halogen- oder cyanosubstituierten C₁-C₃₀-Kohlenwasserstoffrest, in dem eine oder mehrere, einander nicht benachbarte Methyleneinheiten durch Gruppen -O-, -CO-, -COO-, -OCO-, oder -OCOO-, -S-, -NH- oder -N-C₁-C₂₀-Alkyl ersetzt sein können und in denen eine oder mehrere, einander nicht benachbarte Methineinheiten durch Gruppen, -N=, -N=N-, oder -P= ersetzt sein können, und
- **M**: Wasserstoff, Alkali-, Erdalkalimetallion oder Ammoniumion bedeuten,
wobei jeweils 2 Reste, die ausgewählt werden aus den Paaren **R**^{**1**} und **R**^{**2**}, **R**^{**3**} und **R**^{**4**} sowie **R**^{**1**} und **R**^{**3**}, miteinander verknüpft sein können.

Bei dem Verfahren handelt es sich um eine Retroaldolisierung. Nach dem Verfahren lassen sich Carbonylverbindungen der allgemeinen Formel (2) in sehr hohen Ausbeuten von bis zu > 90% und sehr hohen Reinheiten einfach und bei gleichzeitig sehr guten Raum-Zeit-Ausbeuten und damit hoher Wirtschaftlichkeit gewinnen.

Die C₁-C₃₀-Kohlenwasserstoffreste für **R**^{**1**}**, R**^{**2**}**, R**^{**3**} und **R**^{**4**} sind vorzugsweise lineare, verzweigte oder cyclische C₁-C₂₀-Alkyl-, C₃-C₂₀-Alkoxycarbonylalkyl-, C₂-C₂₀-Alkenyl-, C₅-C₂₀-Acetalalkenyl-, C₃-C₂₀-Alkylcarbonyloxyalkylreste, die substituiert sein können durch F, Cl, Br, J, CN und C₁-C₈-Alkoxyreste und in denen Methyleneinheiten durch -O-, -S-, -NH-, -N-C₁-C₂₀-Alkyl ersetzt sein können; Aryl-, Aralkyl-, Alkaryl-, Aralkenyl-, Alkenylarylreste, in denen eine oder mehrere Methineinheiten durch Gruppen, -N=, -N=N-, -P=, und Methyleneinheiten durch -O-, -S-, -NH-, -N-C₁-C₂₀-Alkyl ersetzt sein können und die substituiert sein können durch F, Cl, Br, J, CN, und C₁-C₁₀-Alkoxyreste und am Ring durch C₁-C₁₀-Alkylreste.

Die Halogenreste **R**^{**3**} und **R**^{**4**} sind vorzugsweise F und Cl.

Die C₁-C₂₀-Alkylreste für -N-C₁-C₂₀-Alkyl bei den Bedeutungen von **R**^{**x**} können verzweigt, unverzweigt oder cyclisch sein. Bevorzugt sind lineare C₁-C₁₀-Alkylreste.

Die β-Hydroxycarbonsäuren der allgemeinen Formel (2) oder deren Salze werden als Flüssigkeiten oder Feststoffe eingesetzt, gegebenenfalls gelöst in nicht-hydroxylischem Lösungsmittel.

Als nicht-hydroxylische Lösungsmittel sind aprotische Lösungsmittel, insbesondere Ether, Kohlenwasserstoffe und Kohlenwasserstoff-substituierte Silane und Siloxane bevorzugt. Als Ether eignen sich Mono- und Polyether, vorzugsweise symmetrische und unsymmetrische Di-C₁-C₁₀-Kohlenwasserstoffether, beispielsweise Dibutylether, Tetrahydrofuran, Dihexylether, Diphenylether, Anisol, Phenetol oder cyclische Ether, wie Cumaron und Tetrahydrofuran. Beispiele für Polyether sind Polyethylenglykoldimethylether und Polyethylenglykoldiethylether.
Als aromatische oder aliphatische Kohlenwasserstoffe sind C₁-C₂₀-Kohlenwasserstoffe und deren Gemische bevorzugt, wie Toluol, Ethylbenzol, Propylbenzol, Isopropylbenzol, Butylbenzol, Xylol, Xylol-Isomerengemisch, Trimethylbenzol, Octan, Isooctan, Nonan, Nonan-Fraktion, Cycloheptan, Cyclooctan, Dimethylcyclohexan, Ethylcyclohexan, Propylcyclohexan, Butylcyclohexan, Petroleumbenzin und Paraffin.
Kohlenwasserstoff-substituierte Silane und Siloxane sind Kohlenwasserstoffe, in denen eine oder mehrere Methylengruppen auch durch Dialkylsilyl- oder Dialkylsiloxygruppen ersetzt sein können. Bevorzugte Beispiele sind Tetraethylsilan, Tetrapropylsilan, Tetrabutylsilan, Dimethyldiphenylsilan und Polydimethylsiloxan.
Lösungsmittel oder Lösungsmittelgemische mit einem Siedepunkt bzw. Siedebereich von bis zu 250° C bei 0,1 MPa sind bevorzugt.

Als Brönstedt-Basen besonders geeignet sind die Hydroxide, Methanolate, Ethanolate, Oxide, Amide und Hydride von Lithium, Natrium, Kalium, Magnesium und Calcium. Bevorzugt werden Alkali- und Erdalkalihydroxide, insbesondere Natrium- und Kaliumhydroxid eingesetzt. Die festen Alkali- und Erdalkalibasen werden in Form von Plätzchen, Schuppen, Kugeln, Perlen, Prills, Microprills oder pulverförmig, bevorzugt pulverförmig und als Prills oder Microprills eingesetzt.

Das Verfahren wird vorzugsweise bei Temperaturen von 50 bis 300°C insbesondere bei 100 bis 250°C durchgeführt. Während der Umsetzung hält man die Temperatur auf dem vorgegebenen Wert. Gegebenenfalls kann die obere Temperaturgrenze durch den Siedepunkt des eingesetzten inerten Lösungsmittels, wie z. B. Di-n-butylether (Kp.: 140-143 °C), Nonan-Fraktion (Kp.: 148-153 °C) oder Xylol-Isomerengemisch (Kp.: 137-143 °C) begrenzt sein.

Das erfindungsgemäße Verfahren kann beispielsweise durchgeführt werden, indem entweder die Mischung aus Base, Hydroxycarbonsäure der allgemeinen Formel (2) und Lösungsmittel auf die gewünschte Temperatur erhitzt wird oder die Mischung aus Base und Lösungsmittel wird zuerst auf die gewünschte Temperatur erhitzt und anschließend wird die Hydroxycarbonsäure der allgemeinen Formel (2) zugegeben. Man kann auch zuerst die Hydroxycarbonsäure der allgemeinen Formel (2) und Lösungsmittel auf die gewünschte Temperatur erhitzen und dann die Base zusetzen. Die durch Spaltung der Hydroxycarbonsäure gebildete Carbonylverbindung der allgemeinen Formel (1) wird anschließend isoliert.

Die Hydroxycarbonsäure der allgemeinen Formel (2) kann auch in Form ihres Alkalimetall-, Erdalkalimetall- oder Ammoniumsalzes eingesetzt werden.

Die gebildete Carbonylverbindung der allgemeinen Formel (1) kann bevorzugt durch Destillation, Extraktion, Kristallisation oder mittels chromatographischer Methoden gewonnen und isoliert werden.

Zur Gewinnung und Isolierung der gebildeten Carbonylverbindungen der allgemeinen Formel (1) wird das Reaktionsgemisch nach beendeter Reaktion und gegebenenfalls Abkühlung auf Temperaturen von 0 bis 150 °C mit Wasser oder einer Mineralsäure, in konzentrierter Form oder in Form einer verdünnten wäßrigen Lösung, und gegebenenfalls einem mit Wasser nicht mischbaren organischen Lösungsmittel zur besseren Phasentrennung versetzt und die Carbonylverbindung durch Destillation, Extraktion, Kristallisation oder mittels chromatographischer Methoden gewonnen und isoliert. Alternativ dazu kann man auch das Reaktionsgemisch zu einer Säure zusetzen (inverse Neutralisation). Es ist auch möglich, die Carbonylverbindungen der allgemeinen Formel (1) nach beendeter Reaktion durch Destillation, Extraktion, Kristallisation oder mittels chromatographischer Methoden direkt aus der Reaktionsmischung zu gewinnen und zu isolieren ohne die Reaktionsmischung gegebenenfalls wäßrig aufzuarbeiten.

Bei der Isolation der Carbonylverbindungen der allgemeinen Formel (1) kann das eingesetzte Lösungsmittel in den meisten Fällen nahezu quantitativ zurückgewonnen werden, was das Verfahren insbesondere bei Durchführung im technischen Maßstab sehr wirtschaftlich macht.

Man kann die Spaltung der Hydroxycarbonsäuren der allgemeinen Formel (2) auch in Gegenwart eines hochsiedenden Lösungsmittels durchführen und die während der Reaktion durch Spaltung gebildeten, niedriger siedenden Carbonylverbindungen der allgemeinen Formel (1) unmittelbar z. B. durch Destillation, gegebenenfalls zusammen mit einem Schleppmittel und gegebenenfalls unter reduziertem Druck von 0,01 bis 1000 mbar, auffangen und isolieren.

Es hat sich bewährt, die Hydroxycarbonsäure der allgemeinen Formel (2) mit der Base im Molverhältnis 1 : (1 bis 10), insbesondere 1 : (1 bis 3) umzusetzen. Die Konzentration der Hydroxycarbonsäure der allgemeinen Formel (2) in nicht-hydroxylischem Lösungsmittel beträgt üblicherweise 5 bis 90 Gewichts-%, insbesondere 10 bis 70 Gewichts-%, bevorzugt 15 bis 50 Gewichts-%.

Der Druckbereich der Reaktion ist unkritisch und kann innerhalb weiter Grenzen variiert werden. Der Druck beträgt üblicherweise 0,00001 bis 20 bar, bevorzugt wird die Reaktion bei 0,0001 bar bis Normaldruck (Atmosphärendruck) durchgeführt.

Die Reaktion kann kontinuierlich oder diskontinuierlich durchgeführt werden. Eine kontinuierliche Reaktionsführung ist insbesondere bei Verwendung eines hochsiedenden Lösungsmittels möglich, wobei die während der Reaktion durch Spaltung gebildeten, niedriger siedenden Carbonylverbindungen der allgemeinen Formel (1) unmittelbar z. B. durch Destillation, gegebenenfalls unter reduziertem Druck bei vorzugsweise 0.01 bis 1000 mbar, aufgefangen und isoliert werden. Bei kontinuierlicher Reaktionsführung kann die Hydroxycarbonsäure der allgemeinen Formel (2) fortwährend zudosiert werden. Bei kontinuierlicher Reaktionsführung wird die Base im Überschuß eingesetzt.

Der Vorteil des erfindungsgemäßen Verfahrens gegenüber den vorbekannten Methoden besteht darin, daß die Spaltung der Hydroxycarbonsäuren der allgemeinen Formel (2) in Gegenwart der vorstehend genannten Basen und insbesondere eines nichthydroxylischen Lösungsmittels mit hohem Umsatz, in vielen Fällen nahezu quantitativ und deshalb mit hohen Ausbeuten bei gleichzeitig sehr hoher Reinheit der isolierten Carbonylverbindungen der allgemeinen Formel (1) sowie in den meisten Fällen in unerwartet kurzen Reaktionszeiten von 30 min bis 4 h überaus rasch verläuft, was die Reaktion insbesondere bei Durchführung im technischen Maßstab aufgrund sehr hoher Raum-Zeitausbeuten sehr wirtschaftlich macht.
Zudem verläuft die Spaltung der Hydroxycarbonsäuren der allgemeinen Formel (2) in den meisten Fällen mit sehr hohen Ausbeuten der isolierten Carbonylverbindungen der allgemeinen Formel (1) bei nur geringem Überschuß der eingesetzten Base von 1,5 bis 3 Äquivalenten bezogen auf die Hydroxycarbonsäure, was die Reaktion insbesondere bei Durchführung im technischen Maßstab sehr wirtschaftlich macht.

Es war nicht vorherzusehen, dass die Spaltung der Hydroxycarbonsäuren der allgemeinen Formel (2) unter den erfindungsgemäßen Bedingungen, insbesondere bei Verwendung eines nichthydroxylischen Lösungsmittels sehr rasch und mit sehr hohem Umsatz verläuft und die Carbonylverbindungen der allgemeinen Formel (1) in sehr hohen Ausbeuten isoliert werden können.

Alle vorstehenden Symbole der vorstehenden Formeln weisen ihre Bedeutungen jeweils unabhängig voneinander auf.

In den folgenden Beispielen sind, falls jeweils nicht anders angegeben, alle Mengen- und Prozentangaben auf das Gewicht bezogen, alle Drücke 0,10 MPa (abs.) und alle Temperaturen 20°C.

### Beispiel 1: Herstellung von 1-Phenylhexan-3-on

Bei Raumtemperatur wurden in einem Dreihalskolben mit Rückflußkühler, Innenthermometer und Rührer 6,8 g Natriumhydroxid-Prills (170 mmol) in 100 ml Nonan-Fraktion (Kp.: 148-153 °C) vorgelegt. Nachdem 20 g 3-Hydroxy-3-(2'phenylethyl)hexansäure (85 mmol), hergestellt nach K. S. Fors, J. R. Gage, R. F. Heier, R. C. Kelly, W. R. Perrault, N. Wicnienski, J. Org. Chem. 63, 7348 (1998), zugefügt wurden, erwärmte man die Mischung auf 155 °C, wobei sich zuerst eine klare Lösung bildete. Die Mischung wurde 2 h bei 150 °C gerührt, wobei sich ein farbloser Niederschlag bildete. Man ließ dann auf 20 °C abkühlen, hydrolysierte mit 80 ml 2 N Salzsäure (pH-Wert: 1), rührte die Mischung 15 min und trennte die organische Phase ab. Die organische Phase wurde abschließend mit 30 ml gesättigter Natriumhydrogencarbonat-Lösung gewaschen. Nach Phasentrennung wurde über Natriumsulfat getrocknet und das Lösungsmittel im Vakuum abdestilliert (das Lösungsmittel wurde zu > 90 % zurückgewonnen). 1-Phenylhexan-3-on wurde in einer Ausbeute von 13,6 g (91 % d. Th.) mit einem Siedepunkt von 134 °C (13 mbar) erhalten.

Analoge Herstellung in den Lösungsmitteln Di-n-butylether (Kp. 140-143 °C) und Xylol-Isomerengemisch (Kp.: 137-143 °C) lieferte 1-Phenyl-hexan-3-on in Ausbeuten von 12,5 g und 13,3 g (83 bzw. 89 % d. Th.).

### Beispiel 2: Herstellung von 1-Phenylhexan-3-on

Bei Raumtemperatur wurden in einem Dreihalskolben mit Tropftrichter, Innenthermometer und Rührer 8,5 g Natriumhydroxid-Prills (213 mmol) in 100 ml Polyethylenglykol 1000 dimethylether (Smp.: 42 °C) vorgelegt. Nachdem 25 g 3-Hydroxy-3-(2'-phenylethyl)hexansäure (106 mmol), hergestellt nach K. S. Fors, J. R. Gage, R. F. Heier, R. C. Kelly, W. R. Perrault, N. Wicnienski, J. Org. Chem. 63, 7348 (1998), zugefügt wurden, erwärmte man die Mischung 10 min auf 80 °C und dann auf 150 °C. Anschließend wurde der Druck auf 2,5 mbar reduziert und die Mischung 2 h bei 150 °C und einem Druck von 2,5 mbar gerührt, wobei durch Spaltung gebildetes 1-Phenylhexan-3-on in einer gekühlten Vorlage aufgefangen wurde. Das in der Vorlage aufgefangene 1-Phenylhexan-3-on wurde nach Waschen mit 20 ml Wasser in einer Ausbeute von 12,1 g (81 % d. Th.) mit einem Siedepunkt von 133 °C (13 mbar) erhalten.

### Beispiel 3: (Vergleichsbeispiel)

Herstellung von 1-Phenylhexan-3-on nach D. Ivanoff, Bull. Soc. Chim. France 36, 321 (1933) und C. S. Rondestvedt, M. E. Rowley, J. Am. Chem. Soc. 78, 3804 (1956)

Bei Raumtemperatur wurden in einem Dreihalskolben mit Rückflußkühler, Innenthermometer und Rührer 6,8 g Natriumhydroxid-Prills (170 mmol) in 100 ml 1-Butanol vorgelegt. Nachdem 20 g 3-Hydroxy-3-(2'-phenylethyl)hexansäure (85 mmol), hergestellt nach K. S. Fors, J. R. Gage, R. F. Heier, R. C. Kelly, W. R. Perrault, N. Wicnienski, J. Org. Chem. 63, 7348 (1998), zugefügt wurden, erwärmte man die Mischung auf 115 °C. Die Mischung wurde 2 h bei 115 °C gerührt, auf 20 °C abgekühlt, mit 80 ml 2 N Salzsäure hydrolysiert (pH-Wert: 1) und Mischung 15 min gerührt. Nach Phasentrennung wurde die organische Phase mit 30 ml gesättigter Natriumhydrogencarbonat-Lösung gewaschen. Nach Phasentrennung wurde über Natriumsulfat getrocknet und das Lösungsmittel im Vakuum abdestilliert. Der Umsatz an 1-Phenylhexan-3-on beträgt 9 % d. Th..

## Patentansprüche

1. Verfahren zur Herstellung von Carbonylverbindung der allgemeinen Formel (1)
R¹R²C=O (1),
bei dem β-Hydroxycarbonsäure oder deren Salz der allgemeinen Formel (2)
R¹R²C(OH)-CR³R⁴-COOM (2),
in Gegenwart von Brönstedt-Base, die ausgewählt wird aus Hydroxiden, Alkanolaten, Oxiden, Amiden und Hydriden der Alkali- und Erdalkalimetalle und nicht-hydroxylischem Lösungsmittel gespalten wird, wobei
**R**^{**1**} einen gegebenenfalls halogen- oder cyanosubstituierten C₁-C₃₀-Kohlenwasserstoffrest, in dem eine oder mehrere, einander nicht benachbarte Methyleneinheiten durch Gruppen - O-, -CO-, -COO-, -OCO-, oder -OCOO-, -S- oder -N**R**^{**x**}-ersetzt sein können und in denen eine oder mehrere, einander nicht benachbarte Methineinheiten durch Gruppen, -N=,-N=N-,oder -P= ersetzt sein können,
**R**^{**2**} Wasserstoff oder einen gegebenenfalls halogen- oder cyanosubstituierten C₁-C₃₀-Kohlenwasserstoffrest, in dem eine oder mehrere, einander nicht benachbarte Methyleneinheiten durch Gruppen -O-, -CO-, -COO-, -OCO-, oder -OCOO-, -S- oder -N**R**^{**x**}- ersetzt sein können und in denen eine oder mehrere, einander nicht benachbarte Methineinheiten durch Gruppen, -N=,-N=N-,oder -P= ersetzt sein können,
**R**^{**3**} und **R**^{**4**} Wasserstoff, Halogen oder einen gegebenenfalls halogen- oder cyanosubstituierten C₁-C₃₀-Kohlenwasserstoff-rest, in dem eine oder mehrere, einander nicht benachbarte Methyleneinheiten durch Gruppen -O-, -CO-, -COO-, -OCO-, oder -OCOO-, -S- oder -N**R**^{**x**}- ersetzt sein können und in denen eine oder mehrere, einander nicht benachbarte Methineinheiten durch Gruppen, -N=,-N=N-,oder -P= ersetzt sein können,
**R**^{**x**} Wasserstoff oder einen gegebenenfalls halogen- oder cyanosubstituierten C₁-C₃₀-Kohlenwasserstoffrest, in dem eine oder mehrere, einander nicht benachbarte Methyleneinheiten durch Gruppen -O-, -CO-, -COO-, -OCO-, oder -OCOO-, -S-, -NH- oder -N-C₁-C₂₀-Alkyl ersetzt sein können und in denen eine oder mehrere, einander nicht benachbarte Methineinheiten durch Gruppen, -N=, -N=N-, oder -P= ersetzt sein können, und
**M** Wasserstoff, Alkali-, Erdalkalimetallion oder Ammoniumion bedeuten,
wobei jeweils 2 Reste, die ausgewählt werden aus den Paaren **R**^{**1**} und **R**^{**2**}**, R**^{**3**} und **R**^{**4**} sowie **R**^{**1**} und **R**^{**3**}, miteinander verknüpft sein können.

2. Verfahren nach Anspruch 1, bei dem das nicht-hydroxylische Lösungsmittel ausgewählt wird aus Ethern, Kohlenwasserstoffen und Kohlenwasserstoff-substituierten Silanen und Siloxanen.

3. Verfahren nach Anspruch 1 oder 2, bei dem die Brönstedt-Base ausgewählt wird aus Alkali- und Erdalkalihydroxiden.

4. Verfahren nach Anspruch 1 bis 3, bei dem die Hydroxycarbonsäure der allgemeinen Formel (2) mit der Base im Molverhältnis 1 : (1 bis 10) umgesetzt wird.

## Claims

1. Process for the preparation of a carbonyl compound of the general formula (1)
R¹R²C=O (1),
in which a β-hydroxycarboxylic acid or its salt of the general formula (2)
R¹R²C(OH)-CR³R⁴-COOM (2)
is cleaved in the presence of a Brönstedt base. which is selected from hydroxides, alkanolates, oxides, amides and hydrides of the alkali metals and alkaline earth metals and a hydroxyl-free solvent, where
**R**^{**1**} denotes an optionally halogen- or cyano- substituted C₁-C₃₀-hydrocarbon radical in which one or more, nonneighbouring methylene units may be replaced by groups -O-, -CO-, -COO-, -OCO- or - OCOO-, -S- or -N**R**^{**x**}- and in which one or more, nonneighbouring methine units may be replaced by groups -N=, -N=N- or -P=,
**R**^{**2**} denotes hydrogen or an optionally halogen- or cyano-substituted C₁-C₃₀-hydrocarbon radical in which one or more, nonneighbouring methylene units may be replaced by groups -O-, -CO-, -COO-, -OCO- or -OCOO-, -S- or -N**R**^{**x**}- and in which one or more, nonneighbouring methine units may be replaced by groups -N=, -N=N- or -P=,
**R**^{**3**} and **R**^{**4**} denote hydrogen, halogen or an optionally halogen- or cyano-substituted C₁-C₃₀-hydrocarbon radical in which one or more, nonneighbouring methylene units may be replaced by groups -O-, - CO-, -COO-, -OCO- or -OCOO-, -S- or -N**R**^{**x**}- and in which one or more, nonneighbouring methine units may be replaced by groups -N=, -N=N- or -P=,
**R**^{**x**} denotes hydrogen or an optionally halogen- or cyano-substituted C₁-C₃₀-hydrocarbon radical in which one or more, nonneighbouring methylene units may be replaced by groups -O-, -CO-, -COO-, -OCO- or -OCOO-, -S-, -NH- or -N-C₁-C₂₀-alkyl and in which one or more, nonneighbouring methine units may be replaced by groups -N=, -N=N- or -P=, and
**M** denotes hydrogen, an alkali metal ion, an alkaline earth metal ion or an ammonium ion,
where in each case 2 radicals which are selected from the pairs **R**^{**1**} and **R**^{**2**}**, R**^{**3**} and **R**^{**4**} and **R**^{**1**} and **R**^{**3**} can be linked to one another.

2. Process according to Claim 1, in which the hydroxyl-free solvent is selected from ethers, hydrocarbons and hydrocarbon-substituted silanes and siloxanes.

3. Process according to Claim 1 or 2, in which the Brönstedt base is selected from alkali metal and alkaline earth metal hydroxides.

4. Process according to any of Claims 1 to 3, in which the hydroxycarboxylic acid of the general formula (2) is reacted with the base in the molar ratio 1 : (1 to 10).

## Revendications

1. Procédé pour la préparation d'un composé carbonyle de formule générale (1)
R¹R²C=O (1)
dans lequel on dissocie l'acide β-hydroxycarboxylique ou son sel de formule générale (2)
R¹R²C(OH)-CR³R⁴-COOM (2)
en présence d'une base de Brönstedt choisie parmi les hydroxydes, les alcanolates, les oxydes, les amides et les hydrures des métaux alcalins et alcalino-terreux et d'un solvant non-hydroxyle,
R¹ signifiant un radical hydrocarboné en C₁ à C₃₀ le cas échéant substitué par halogène ou cyano, dans lequel une ou plusieurs unités méthylène qui ne sont pas adjacentes peuvent être remplacées par des groupements -O-, -CO-, -COO-, -OCO- ou -OCOO-, -S- ou -NR^{x}- et dans lesquels une ou plusieurs unités méthine qui ne sont pas adjacentes peuvent être remplacées par des groupements -N=, -N=N- ou -P=,
R² signifiant hydrogène ou un radical hydrocarboné en C₁ à C₃₀ le cas échéant substitué par halogène ou cyano, dans lequel une ou plusieurs unités méthylène qui ne sont pas adjacentes peuvent être remplacées par des groupements -O-, -CO-, -COO-, -OCO- ou -OCOO-, -S- ou -NR^{x}- et dans lesquels une ou plusieurs unités méthine qui ne sont pas adjacentes peuvent être remplacées par les groupements -N=, -N=N- ou -P=,
R³ et R⁴ signifiant hydrogène, halogène ou un radical hydrocarboné en C₁ à C₃₀ le cas échéant substitué par halogène ou cyano, dans lequel une ou plusieurs unités méthylène qui ne sont pas adjacentes peuvent être remplacées par des groupements -O-, -CO-, -COO-, -OCO- ou -OCOO-, -S- ou -NR^{x}- et dans lesquels une ou plusieurs unités méthine qui ne sont pas adjacentes peuvent être remplacées par des groupements -N=, -N=N- ou -P=,
R^{x} signifiant hydrogène ou un radical hydrocarboné en C₁ à C₃₀ le cas échéant substitué par halogène ou cyano, dans lequel une ou plusieurs unités méthylène qui ne sont pas adjacentes peuvent être remplacées par des groupements -O-, -CO-, -COO-, -OCO- ou -OCOO-, -S-, - NH- ou -N-(alkyle en C₁ à C₂₀) et dans lesquels une ou plusieurs unités méthine qui ne sont pas adjacentes peuvent être remplacées par des groupements -N=, -N=N- ou -P=, et
M signifiant hydrogène, ion de métal alcalin ou alcalino-terreux ou ion ammonium,
à chaque fois 2 radicaux qui sont choisis parmi les paires R¹ et R², R³ et R⁴ ainsi que R¹ et R³ pouvant être liés l'un avec l'autre.

2. Procédé selon la revendication 1, dans lequel le solvant non-hydroxyle est choisi parmi les éthers, les hydrocarbures et les silanes et siloxanes substitués par hydrocarbure.

3. Procédé selon la revendication 1 ou 2, dans lequel la base de Brönstedt est choisie parmi les hydroxydes de métal alcalin et alcalino-terreux.

4. Procédé selon les revendications 1 à 3, dans lequel l'acide hydroxycarboxylique de formule générale (2) est transformé avec la base dans un rapport molaire 1 : (1 à 10).
